Europäisches Patentamt

European Patent Office

Office européen des brevets

(19)

(11) Veröffentlichungsnummer: **0 189 097**
**B1**

# EUROPÄISCHE PATENTSCHRIFT

(12)

(45) Veröffentlichungstag der Patentschrift:
**01.06.88**

(51) Int. Cl.⁴: **C 07 D 333/32**

(21) Anmeldenummer: **86100425.7**

(22) Anmeldetag: **14.01.86**

(54) Verfahren zur Herstellung von Thiotetronsäure.

(30) Priorität: 16.01.85 CH 193/85

(43) Veröffentlichungstag der Anmeldung:
30.07.86 Patentblatt 86/31

(45) Bekanntmachung des Hinweises auf die Patenterteilung:
01.06.88 Patentblatt 88/22

(84) Benannte Vertragsstaaten:
AT BE CH DE FR GB IT LI LU NL SE

(56) Entgegenhaltungen:
BERICHTE DER DEUTSCHEN CHEMISCHEN
GESELLSCHAFT, Jahrgang 46, Band II, 1913, Seiten
2103-2107, Kommissionsverlag von R. Friedländer &
Sohn, DE; E. BENARY: "Über Thio-tetronsäure und
Derivate"

(73) Patentinhaber: LONZA AG, Gampel/Wallis (CH)

(72) Erfinder: Meul, Thomas, Dr., Balfrinstrasse 21, Visp
(Kanton Wallis) (CH)
Erfinder: Tenud, Leander, Dr. Ing.-Chem.,
Balfrinstrasse 23, Visp (Kanton Wallis) (CH)

(74) Vertreter: Weinhold, Peter, Dr. et al, Patentanwälte Dr. V.
Schmied-Kowarzik Dipl.-Ing. G. Dannenberg Dr. P.
Weinhold Dr. D. Gudel Dipl.-Ing. S. Schubert Dr. P. Barz
Siegfriedstrasse 8, D-8000 München 40 (DE)

ACTORUM AG

## Beschreibung

Die Erfindung betrifft ein neues Verfahren zur Herstellung von Thiotetronsäure.

Thiotetronsäure könnte als Zwischenprodukt zur Herstellung von (±) Thiolactomycin, einem Antibiotikum mit breitem Wirkungsspektrum, eine bedeutende Anwendungsmöglichkeit finden. Tetrahedron Letters Vol 25 No 46 pp 5243-5246, 1984.

Aus E. Benary, Chem. Berichte 46 2 103 (1913) ist bekannt, die Thiotetronsäure ausgehend von Acetylthioglycoylchlorid durch Reaktion mit Natriummalonester und anschliessendem Ringschluss und Wasserbehandlung herzustellen.

D.B. Macierewicz Rocz. chem. 47, 1735 (1973) hat die Reaktion von E. Benary nachvollzogen und dabei Thiotetronsäure in einer Ausbeute von 30,3%, bezogen auf das eingesetzte Acetylthioglycoylchlorid erhalten.

Ein andere Möglichkeit zeigt die Synthese von J.Z. Mortensen et al Tetrahedron 27, 3839 (1971). Ausgehend von 2,4 Dibromthiophen erhält er über 3 Stufen durch Umsetzung mit Butyllithium und t-Butylperbenzoat in einer Ausbeute von 46,2% die Thiotronsäure.

Bei allen diesen herkömmlichen Synthesen sind die Ausbeute für ein technisches Verfahren viel zu bescheiden.

Ausserdem zeichnen sich die Verfahren durch Umständlichkeit, teure Edukte und durch schwierig handhabbare Reagenzien aus.

Die Aufgabe der vorliegenden Erfindung war es daher, ein Verfahren zur Herstellung der Thiotronsäure zu finden, das sich durch hohe Ausbeuten, günstige Edukte und einfache Verfahrensweise auszeichnet.

Dies wird erfindungsgemäss dadurch erreicht, dass 4-Chloracetessigsäurechlorid mit Schwefelwasserstoff in Gegenwart eines Amins als Base behandelt wird.

Zweckmässig wird von Diketen ausgegangen, dass auf bekannte Weise (EP Appl 28 709) durch Chlorieren zum 4-Chloracetessigsäurechlorid umgesetzt wird und dann in situ zur Weiterumsetzung herangezogen werden kann.

Es wird zweckmässig in Lösungsmitteln gearbeitet. Verwendung finden vorteilhaft die halogenierten Kohlenwasserstoffe wie Methylenchlorid oder Chloroform.

Besonders geeignet ist das Methylenchlorid.

Als organisches Amin finden zweckmässig primäre, sekundäre und tertiäre Amine sowie Ammioniak und Guanidine Anwendung. Vorzugsweise kommen Amine mit niedermolekularen Alkylgruppen (d.h. solchen mit 1 bis 6 Kohlenstoffatomen) in Betracht, insbesondere werden tertiäre Amine eingesetzt; besonders bevorzugt wird Triethylamin verwendet.

Der Schwefelwasserstoff wird vorteilhaft gasförmig eingesetzt.

Das Eduktverhältnis in Mol, 4-Chloracetessigsäurechlorid zu Schwefelwasserstoff zu Amin liegt zweckmässig in einem Bereich zwischen 1:2:2 und 1:4:3 vorzugsweise zwischen 1:2.5:2 und 1:3.5:2.2.

Um eine Reaktion des 4-Chloracetessigsäurechlorids mit den Aminen zu 4-Chloracetessigsäureamiden bzw. eine basische Katalytische Dimerisierung zu Dichlordehydracetsäure zu vermeiden, wird zweckmässig so vorgegangen, dass die Säurechlorid Lösung zunächst mit Schwefelwasserstoff und anschliessend mit dem Amin versetzt wird. Ebenso wichtig ist das Verhältnis in Mol von $H_2S$ zu Amin, denn nur bei einem äquimolaren Verhältnis, besser jedoch bei überschüssigem $H_2S$ wird die Bildung der oben erwähnten unerwünschten Nebenprodukte unterdückt. Die Umsetzung des Säurechlorids zu Thiotetronsäure ist unmittelbar nach Zugabe des Amins beendet.

Die Umsetzung des 4-Chloracetessigsäurechlorids wird zweckmässig bei Temperaturen von 0°C bis –40°C vorzugsweise bei –10°C bis –20°C durchgeführt.

Die Aufarbeitung des Reaktionsgemisches kann durch Abtrennen des Lösungsmittels durch Eindampfen oder Destillation und durch anschliessende Extraktion des Zielproduktes aus dem Rückstand mit geeigneten Lösungsmitteln erfolgen.

Geeignete Lösungsmittel sind etherische Lösungsmittel wie THF, Dioxan oder Diethylether.

Besonders vorteilhaft wird Diethylether eingesetzt.

Zweckmässig wird die Extraktlösung vor dem Eindampfen zur Abtrennung geringer Mengen dimerer Anhydrothiotetronsäure mit einem Absorptionsmittel wie Kieselgel, behandelt.

Nach Eindampfen und Trocknen kann die Thiotronsäure als kristallines Produkt erhalten werden.

*Beispiel*

Aus 8,5 g (0,1 mol) Diketen und 7,1 g (0,1 mol) Chlor, gelöst in 100 ml Methylenchlorid stellte man nach bekanntem Verfahren das 4-Chloracetessigsäurechlorid her. Diese Lösung wurde mit 500 ml Methylenchlorid verdünnt und bei –15°C mit gasförmigem Schwefelwasserstoff gesättigt. Zu dieser Lösung tropfte man bei –10°C innerhalb von 1,5 Stunden eine Lösung von 20,2 g (0,2 mol) Triethylamin in 180 ml Methylenchlorid zu. (Eduktverhältnis 4-Chloracetessigsäurechlorid : $H_2S$ : Triethylamin 1:3:2). Man liess die Reaktionslösung auf Raumtemperatur erwärmen, destillierte das Lösungsmittel am Rotationsverdampfer ab und löste die Thiotetronsäure mit 200 ml Ether in einem Soxhlet-Extraktor aus dem festen Rückstand heraus. Diese Etherlösung wurde über eine mit Kieselgel gefüllte Säule filtriert. Man erhielt 7,0 g orangefarbenes, kristallines Produkt mit einem Gehalt (HPLC) von 88,0%. Dies entsprach 6,2 g 100%igem Produkt ≙ 53,4% Ausbeute. Schmelzpunkt: 115°C.

$^1$H-NMR-Spektrum (300 MHz, DMSO-D6): δ = 4,04 (d, 2H, J = 1,0Hz), 5,36 (t, 1H), 12,55 (verbr. s, 1H).

## Patentansprüche

1. Verfahren zur Herstellung von Thiotetronsäure, dadurch gekennzeichnet, dass 4-Chloracetessigsäurechlorid mit Schwefelwasserstoff in Gegenwart eines Amins umgesetzt wird.

2. Verfahren nach Patentanspruch 1, dadurch gekennzeichnet, dass als Amin primäre, sekundäre oder tertiäre Amine, Ammoniak oder Guanidine angewendet werden.

3. Verfahren nach Patentansprüchen 1 und 2, dadurch gekennzeichnet, dass tertiäre Amine angewendet werden.

4. Verfahren nach Patentansprüchen 1 bis 3, dadurch gekennzeichnet, dass in Lösungsmitteln gearbeitet wird.

5. Verfahren nach Patentanprüchen 1 bis 4, dadurch gekennzeichnet, dass als Lösungmittel halogenierte Kohlenwasserstoffe verwendet werden.

6. Verfahren nach Patentansprüchen 1 bis 5, dadurch gekennzeichnet, dass als Verhältnis von Schwefelwasserstoff zu Amin wenigstens ein äquimolekulares Verhältnis, vorzugsweise jedoch ein Überschuss an Schwefelwasserstoff verwendet wird.

7. Verfahren nach Patentansprüchen 1 bis 6, dadurch gekennzeichnet, dass das Eduktverhältnis in Mol 4-Chloracetessigsäurechlorid zu Schwefelwasserstoff zu Amin zwischen 1:2:2 und 1:4:3 liegt.

8. Verfahren nach Patentansprüchen 1 bis 7, dadurch gekennzeichnet, dass die Umsetzung bei Temperaturen zwischen 0°C und –40°C durchgeführt wird.

## Claims

1. Process for the preparation of thiotetronic acid, characterized in that 4-chloracetoacetic acid chloride is reacted with hydrogen sulfide in the presence of an amine.

2. Process according to claim 1, characterized in that as amine primary, secondary or tertiary amines, ammonia or guanidines are used.

3. Process according to claims 1 and 2, characterized in that tertiary amines are used.

4. Process according to claim 1 to 3, characterized in that one operates in solvents.

5. Process according to claim 1 to 4, chartacterized in that as solvents halogenated hydrocarbons are used.

6. Process according to claim 1 to 5, characterized in that as ratio of hydrogen sulfide to amine at leat an equimolar ratio, preferably however an excess of hydrogen sulfide, is used.

7. Process according to claims 1 to 6, characterized in that the educt ratio in moles of 4-chloroacetoacetic acid chloride to hydrogen sulfide to amine is between 1:2:2 and 1:4:3.

8. Process according to claims 1 to 7, characterized in that the reaction is carried out at temperature between 0 and –40°C.

## Revendications

1. Procédé pour la préparation de l'acide thiotétronique, caractérisé en ce que l'on fait réagir le chlorure de l'acide 4-chloro-acétylacétique avec du sulfure d'hydrogène, en présence d'une amine.

2. Procédé selon la revendication 1, caractérisé en ce que l'on utilise comme amine, des amines primaires, secondaires ou tertiaires, de l'ammoniac ou de la guanidine.

3. Procédé selon les revendications 1 et 2, caractérisé en ce que l'on utilise des amines tertiaires.

4. Procédé selon les revendications 1 à 3, caractérisé en ce que l'on travaille dans des solvants.

5. Procédé selon les revendications 1 à 4, caractérisé en ce que l'on utilise comme solvants des hydrocarbures halogénés.

6. Procédé selon les revendications 1 à 5, caractérisé en ce que l'on utilise comme rapport de l'hydrogène sulfuré à l'amine au moins un rapport équimoléculaire, de préférence toutefois un excès d'hydrogène sulfuré.

7. Procédé selon les revendications 1 à 6, caractérisé en ce que la proportion en moles des produits de départ, chlorure de l'acide 4-chloro-acétylacétique à l'hydrogène sulfuré à l'amine, se situe entre 1:2:2 et 1:4:3.

8. Procédé selon les revendications 1 à 7, caractérisé en ce que la réaction est effectuée à des températures entre 0° et –40°C.